# EUROPEAN PATENT SPECIFICATION

(11) **EP 2 726 078 B1**
(45) Date of publication and mention of the grant of the patent: **09.08.2017**
(21) Application number: 12731413.6
(22) Date of filing: 27.06.2012
(51) Int. Cl.: A61K 31/53, A61P 25/00, A61P 37/00, A61P 29/00

(54) **METHODS FOR TREATING IMMUNE-MEDIATED DISEASES OF THE NERVOUS SYSTEM ADMINISTERING A COMPOUND COMPRISING AGENTS THAT INHIBIT PROKINETICIN RECEPTORS ACTIVITY**
VERFAHREN ZUR BEHANDLUNG VON IMMUNVERMITTELTEN ERKRANKUNGEN DES NERVENSYSTEMS MITTELS VERABREICHUNG EINER VERBINDUNG MIT WIRKSTOFFEN ZUR HEMMUNG DER WIRKUNG VON PROKINETICIN-REZEPTOREN
MÉTHODES DE TRAITEMENT DE MALADIES IMMUNITAIRES DU SYSTÈME NERVEUX PAR L'ADMINISTRATION D'UN COMPOSÉ COMPRENANT DES AGENTS QUI INHIBENT L'ACTIVITÉ DES RÉCEPTEURS DE PROKINÉTICINE

(30) Priority: 30.06.2011 IT MI20111225
(43) Date of publication of application: 07.05.2014
(73) Proprietor: Fondazione I.R.C.C.S. Istituto Neurologico 'Carlo Besta', 20123 Milano (IT)
(72) Inventor: PEDOTTI, Rosetta, I-20133 Milano (IT); ABOU HAMDAN, Mohamad, I-10141 Torino (IT)
(74) Representative: Ferreccio, Rinaldo
(86) International application number: PCT/EP2012/062511
(87) International publication number: WO 2013/000974

(56) References cited:
- WO-A2-2006/102112
- US-A1- 2009 306 076
- PEDOTTI R ET AL: "An unexpected version of horror autotoxicus: anaphylactic shock to a self-peptide.", NATURE IMMUNOLOGY MAR 2001 LNKD- PUBMED:11224520, vol. 2, no. 3, March 2001 (2001-03), pages 216-222, XP002658856, ISSN: 1529-2908
- KUERTEN ET AL: "Comparing the CNS morphology and immunobiology of different EAE models in C57BL/6 mice - A step towards understanding the complexity of multiple sclerosis", ANNALS OF ANATOMY, JENA, DE, vol. 190, no. 1, 4 February 2008 (2008-02-04), pages 1-15, XP022450122, ISSN: 0940-9602, DOI: 10.1016/J.AANAT.2007.11.001
- GIANFRANCO BALBONI ET AL: "Triazine Compounds as Antagonists at Bv8-Prokineticin Receptors", JOURNAL OF MEDICINAL CHEMISTRY, AMERICAN CHEMICAL SOCIETY, US, vol. 51, no. 23, 11 December 2008 (2008-12-11), pages 7635-7639, XP002658188, ISSN: 0022-2623, DOI: 10.1021/JM800854E [retrieved on 2008-11-12]

## Description

The present invention relates to a compound comprising non-peptide prokineticin receptors antagonists for use in the treatment of immune-mediated diseases of the central nervous system, peripheral nervous system and neuromuscular junctions.

Prokineticins are small secreted bioactive peptides highly conserved from reptiles to human.

Two molecules of this family have been so far described, which are prokineticin 1 (also called EG-VEGF, herein referred to as PK-1) and prokineticin 2 (also called Bv8, herein referred to as PK-2). These two secreted proteins from the AVIT secreted protein family exert their biological effects by stimulating two closely related G-protein-coupled receptors: PK-R1 and PK-R2. Prokineticins and their receptors are widely distributed in mammalian tissues and cells including neurons, granulocytes, macrophages and endothelial cells (Negri et al., life Sci 2007).

The list of biological activities associated with these peptides is rapidly growing. PK1 and PK2 with their receptors are involved in a wide variety of biological functions including gastrointenstinal motility, angiogenesis, neurogenesis, circadian rhythm, reproduction, haematopoiesis, and pain (Negri et al., life Sci 2007; Martucci et al., Br J Pharmacol 2006). They play also an important role in the immune response.

The activation of PK-R1 and PK-R2 on immune cells such as neutrophils, macrophages and dendritic cells promotes chemotaxis and the release of cytokines, whereas in the endothelial cells of capillaries it stimulates angiogenesis. It has been demonstrated that PK-2 induces a pro-inflammatory phenotype in macrophages, since it activates these cells, promoting the migration thereof and the production of pro-inflammatory cytokines like IL-1 and IL-12, while decreasing the levels of the anti-inflammatory cytokine IL-10 (Martucci et al., Br J Pharmacol 2006). PK-2 also modifies the functionality of T lymphocytes by inducing a shift in the Thl/Th2 balance toward a Th1-type response (Franchi et al., 2008). These effects seem to depend on the activation of PK-R1, since they do not manifest themselves in the lymphocytes or macrophages of mice genetically lacking this receptor.

Among the diseases of the central nervous system, one of the most important is multiple sclerosis (MS).

Multiple sclerosis is an autoimmune disease characterized by an immune system attack against the myelin sheath that covers neuronal axons. In Italy there are about 60,000 people affected by MS, with a ratio of approximately 1:1,000 inhabitants. Although it is by now an accredited hypothesis that mechanisms of an immuno-mediated type are at the basis of this disease, the etiopathogenesis of MS has not yet been completely clarified, and the effectiveness of the current therapies is only partly satisfactory. It is well known that in MS and its experimental model, experimental autoimmune encephalomyelitis (EAE), the responses mediated by T-helper (Th) lymphocytes of the types Th1 (characterized by the secretion of interferon-gamma) and Th17 (characterized by the secretion of interleukin 17) play a promoting role, whereas Th2-type responses (characterized by the secretion of interleukin 4, 5 and 13) play a protective role (Steinman, J Ex Med 2008; Liblau et al., Immunology today 1995). In fact, immunomodulating treatments that induce a shift in immune responses from the Th1 type to the Th2 type have demonstrated to be effective both in EAE and in MS. Glatiramer acetate (Copaxone), one of the immunomodulating treatments commonly used in this disease, induces a Th1 to Th2 shift and has demonstrated to be effective in improving the clinical expression of MS (understood as a reduction in relapses of disease and an increase in the interval of time between the first clinical symptoms of MS - clinically isolated syndrome or CIS - and the clinically definite disease).

The present invention is based on the particular discovery that the pathways associated with prokineticins and their receptors PK-R1 and PK-R2 favours the development of multiple sclerosis (MS) in the experimental model thereof (EAE).

Since prokineticins and their receptors are present in cells of both the nervous system and the immune system, where they are hyper-expressed in situations of inflammation, these mediators seem to be the ideal candidates for modulating the neuroimmunological interactions in different inflammatory states of the central and peripheral nervous system.

As MS is a disease associated with a dysregulation of the immune response, it has been hypothesized that if prokineticins have a role in this disease, blocking PK-Rs with an antagonist may reduce the severity of the disease.

The technical task that the present invention has set itself, therefore, is to provide a compound for a new therapeutic use as an agent that can prevent or treat immune-mediated diseases of the nervous system and more specifically multiple sclerosis.

The technical task, as well as these and other objects, according to the present invention, are achieved by providing a compound for a new therapeutic use as prokineticin receptors antagonists in accordance with claim 1.

Other features of the present invention are defined, moreover, in the subsequent claims.

Additional features and advantages of the invention will be more apparent from the description that follows, illustrated by way of non-restrictive example in the appended drawings, in which:
figure 1 shows the structure of non-peptide prokineticin receptors antagonists PC1 and PC7, both effective in reducing the severity of disease in two experimental models of MS: the first experimental model is relapsing-remitting EAE (RR-EAE) and the second model is chronic EAE.
figure 2 shows the modulation of the mRNA expression of PK-2, PK-R1 and PK-R2 in lymph node cells or CD4+ T cells during chronic, MOG₃₅₋₅₅ induced EAE in C57B1/6 mice;
figure 3 shows the levels of PK-2 in the serum of mice with chronic MOG₃₅₋₅₅ induced EAE;
figure 4 shows the reduction in severity of relapsing-remitting PLP₁₃₉₋₁₅₁ induced EAE in SJL mice treated with the prokineticin receptors antagonist PC1, administered in a preventive treatment regimen;
figure 5 shows the reduction in the T-cell response in the splenocytes of mice with relapsing-remitting PLP₁₃₉₋₁₅₁ induced EAE treated with the prokineticin receptors antagonist PC1 starting at the day of the induction of the relapsing-remitting EAE;
figure 6 shows the reduction in severity of relapsing-remitting PLP₁₃₉₋₁₅₁ induced EAE in SJL mice treated with the prokineticin receptors antagonist PC7, administered in a preventive treatment regimen;
figure 7 shows the reduction in severity of relapsing-remitting PLP₁₃₉₋₁₅₁ induced EAE in SJL mice treated with the prokineticin receptors antagonist PC7, administered in a therapeutic treatment regimen;
figure 8 shows the reduction of T-cell response in the lymph nodes of mice with relapsing-remitting PLP₁₃₉₋₁₅₁ induced EAE treated with the prokineticin receptors antagonist PC7 in a preventive treatment regimen;
figure 9 shows the reduction in severity of chronic MOG₃₅₋₅₅ induced EAE in C57B1/6 mice treated with the prokineticin receptors antagonist PC7, administered in a preventive treatment regimen;
figure 10 shows the reduction in severity of chronic MOG₃₅₋₅₅ induced EAE in C57B1/6 mice treated with the prokineticin receptors antagonist PC7, administered in a therapeutic treatment regimen;
figure 11 shows the reduced T-cell response in the lymph nodes of mice with chronic MOG₃₅₋₅₅ induced EAE treated with the prokineticin receptors antagonist PC-7 in a preventive treatment regimen;

According to the invention, use is made of non-peptide prokineticin receptors antagonists, capable of modulating or blocking the signalling of prokineticin receptors, having a basic chemical structure PC1 or PC7, for the treatment of immune-mediated diseases of the central and peripheral nervous system, wherein:

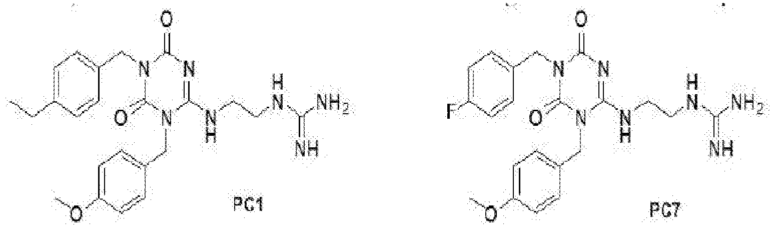

Said prokineticin antagonists (hence antagonists of the known prokineticin receptors, which are receptors 1 and 2, also called PK-R1 and PK-R2, and of any receptors of these molecules that should be discovered in the future) can be advantageously used: for the treatment of multiple sclerosis and other autoimmune diseases or dysimmune-based diseases or diseases with a dysimmune or autoimmune component affecting the central nervous system (including clinically isolated syndrome or CIS, acute demyelinating encephalomyelitis or ADEM, postinfectious encephalomyelitis, Rasmussen's encephalitis, Bickerstaff's encephalitis, paraneoplastic encephalopathies, Alzheimer's disease, Parkinson's disease amyotrophic lateral sclerosis, epilepsy); for the treatment of autoimmune or dysimmune-based diseases or diseases with a dysimmune or autoimmune component affecting the peripheral nervous system and the neuromuscular junction (such as Guillain-Barré syndrome, chronic inflammatory demyelinating polyneuropathy or CIDP, multifocal motor neuropathies or MMN, neuropathy associated with monoclonal components, neuropathy associated with anti-MAG gammopathy; myasthenia gravis; Lambert-Eaton syndrome and Stiff Man Syndrome);
The non-peptide antagonists PC1 and PC7 preferentially bind to prokineticin receptor 1 (PK-R1).

Since prokineticins are capable of promoting Th1-type responses which contribute to the development of MS, it has shown to be useful to intervene in the prokineticin system with the aim of reducing the Th1-type responses directed against myelin and promoting instead Th2-type protective responses to reduce the severity of MS in the experimental models EAE thereof.

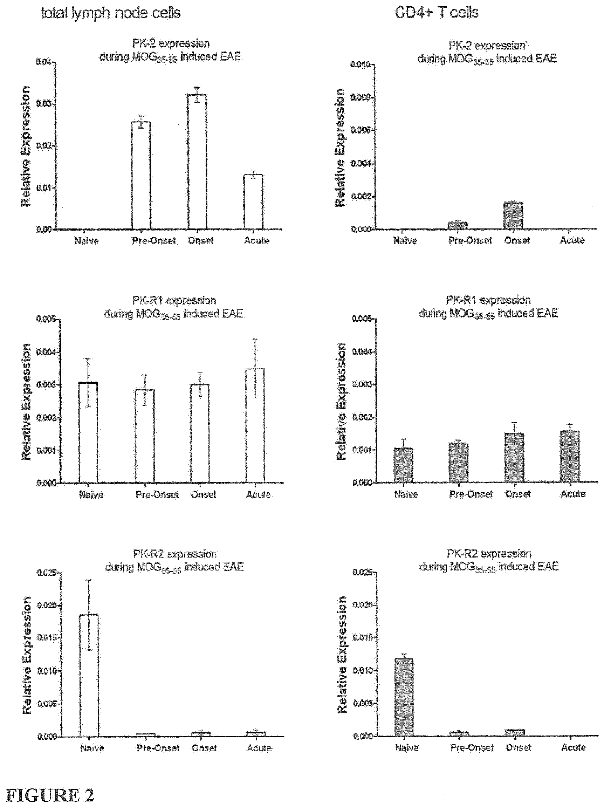

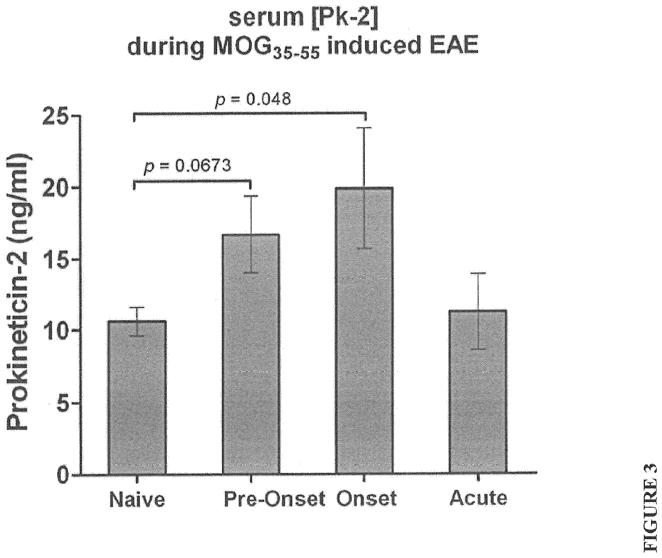

In the study conducted we verified the expression of PK-2 and its receptors PK-R1 and PK-R2 in the experimental model of chronic EAE, an experimental model of MS. The chronic EAE model was obtained in C57B1/6 mice by immunization with myelin oligodendrocyte glycoprotein peptide fragment 35-55 (MOG₃₅₋₅₅) and PTX (Pertussis toxin) [Pedotti et al., PNAS 2003]. During chronic EAE, we observed an increase of mRNA expression of PK-2 by lymph node cells and purified CD4+ T cells in C57B1/6 mice during the disease. This increase of mRNA expression started before the onset of the disease and is less significant after its onset (Figure 2). We observed also an important increase of the levels of the protein PK-2 in the serum of mice with MOG₃₅₋₅₅ induced EAE (Figure 3). This increase of PK-2 was accompanied by a dramatic reduction of PK-R2 expression in lymph node cells and CD4+ T cells. On the other hand, the expression of PK-R1 was not significantly modulated during our model of chronic-EAE. Thus, the couple PK-2/PK-R1 seemed to play an important role during EAE, the experimental model of MS.

To verify if prokineticins and their receptors play a role in EAE, we used two non-peptide antagonists of prokineticin receptors 1 and 2, preferentially blocking receptor 1 (PK-R1), in order to evaluate the effects of the pharmacological blockade of the signalling of these receptors in the clinical expression of two experimental models of MS: the experimental model of relapsing-remitting EAE and the experimental model of chronic EAE.

The relapsing-remitting EAE model was obtained in female SJL mice by immunization with myelin proteolipid protein fragment 139-151 (PLP₁₃₉₋₁₅₁) in complete Freund's adjuvant (CFA) [Pedotti et al., Nat Immunol 2001]. The chronic EAE model was obtained in C57BL/6 mice as mentioned before.

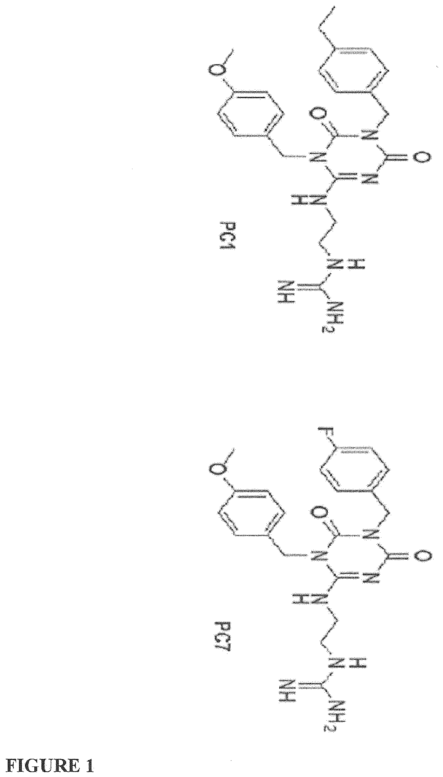

We used non-peptide prokineticin receptors antagonist drugs called PC1 and PC7 (whose chemical structure is shown in Figure 1) to chronically treat the mice starting both from the time of induction of EAE (day 0; preventive treatment) and from the onset of the first symptoms of EAE (day 10-12, approximately; therapeutic treatment).

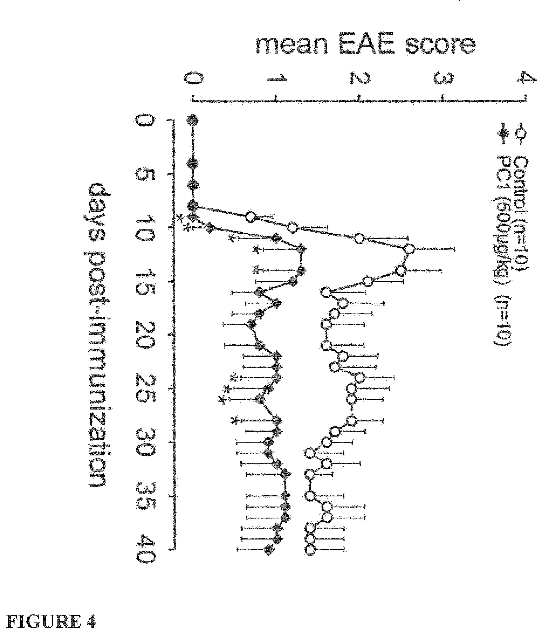

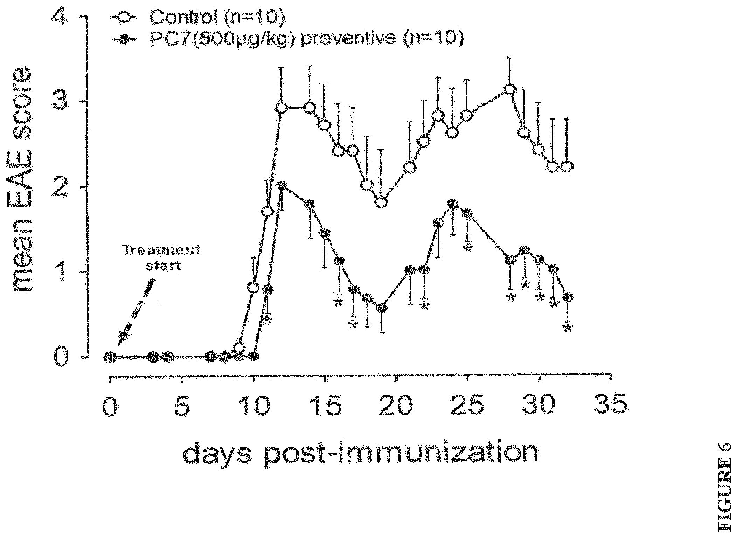

In three consecutive experiments, we observed that the treatment with these drugs significantly improved the clinical expression of relapsing-remitting EAE (by delaying the onset of disease in the mice treated from the time of induction of EAE, and reducing the average severity at the peak of disease (Table 1 and Figure 4, Figure 6).

In particular, the antagonist PC7 demonstrated to be extremely effective in reducing the severity of the disease both in mice with relapsing-remitting EAE, and in mice with chronic EAE, both in the preventive and therapeutic treatment regimens (Table 2, Table 3, Table 4, Table 5, Figure 6, Figure 7, Figure 9 and Figure 10).

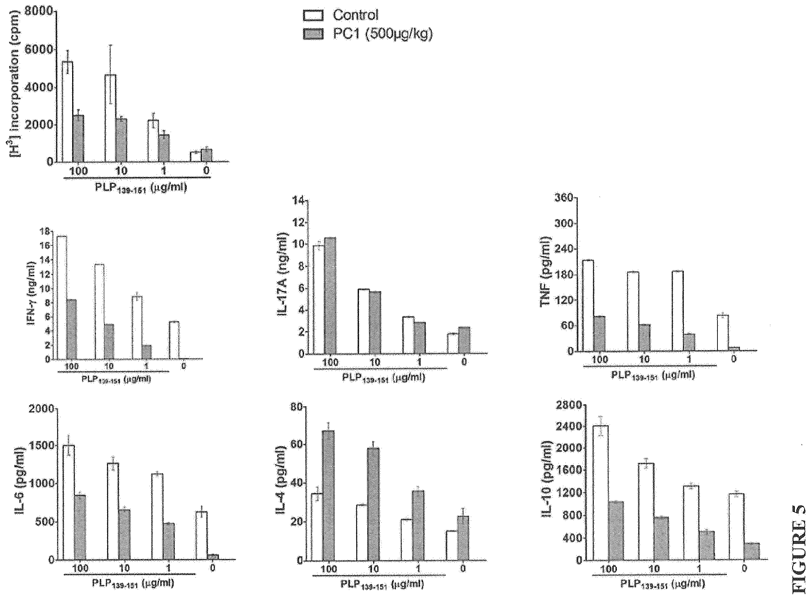

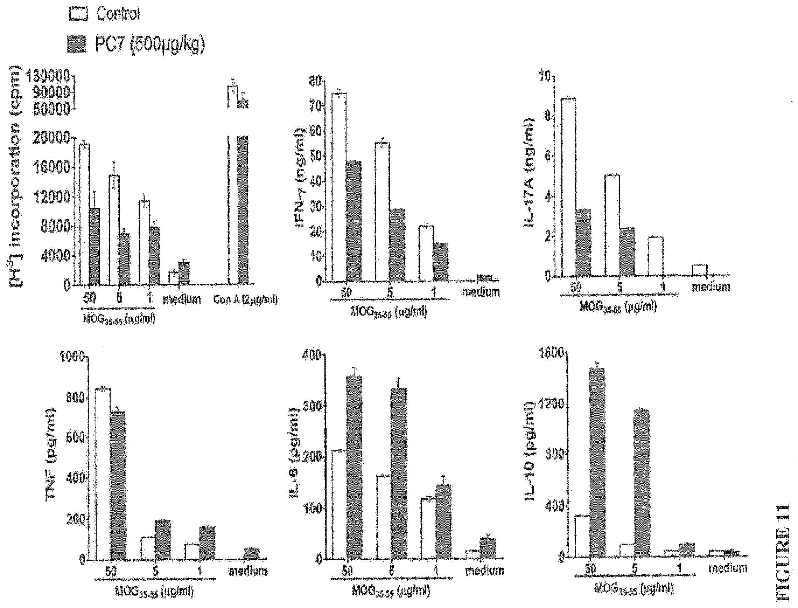

We then conducted functional studies on the lymph node and spleen cells of these treated mice, and observed that in T lymphocytes stimulated with the myelin-specific antigen, PLP₁₃₉₋₁₅₁, the treatment brought a significant reduction in the production of inflammatory Th1 and Th17 cytokines, which are very important for the development of EAE (interleukin [IL]-17, interferon-gamma, and IL-6), while increasing that of anti-inflammatory T-helper 2 cytokines (such as IL-4) (Figure 5, Figure 8, figure 11).

These immunological studies suggest that the treatment with prokineticin receptors inhibitors produced an immunomodulating effect by shifting the specific autoimmune response against myelin from a Th1-type (inflammatory) to a Th2-type (protective) phenotype.

This type of shift has demonstrated to be effective in the treatment of EAE and human multiple sclerosis, so much so that glatiramer acetate (Copaxone), one of the immunomodulating treatments commonly used in this disease, has been demonstrated to induce an analogous Th1 to Th2 shift. The mechanisms of action of prokineticins and their receptors in EAE remain to be clarified. In fact, alongside the already observed ability of these compounds to modify the autoimmune T helper phenotype from Th1 to Th2, other mechanisms could determine the effectiveness of these compounds in EAE, including, for example, an effect of these compounds on the ability of autoreactive T lymphocytes to exit from the lymph nodes and to adhere and penetrate through the inflamed blood-brain barrier (BBB), a crucial step in the development of EAE and human MS. Indeed, several studies indicate that prokineticins and their receptors have an effect on vascular permeability and can favour vessels leakage [Urayama et al., Cardiovasc Res 2009; Guilini et al., Am J Physiol Heart Circ Physiol 2010]. Thus, we can speculate that such mechanism might be involved in the efficacy of these drugs, containing prokineticins-receptors antagonists, in preventing or treating EAE.

It may be noted, in fact, that some of the most recent treatments for MS, such as treatments with Natalizumab (Tysabri) and FTY720 (Fingolimod), are aimed precisely at blocking the trafficking of immune cells toward the target site, the central nervous system.

### EXPERIMENTS CONDUCTED AND RESULTS

### mRNA expression of PK-2, PK-R1 and PK-R2 in mice with MOG35-55 induced EAE (chronic-EAE).

With reference to figure 2, the chronic EAE had been induced in C57B1/6 mice by immunization with MOG₃₅₋₅₅ (100 µg/mouse) and PTX (400 ng/mouse).

Mice were sacrificed at different time points after the immunization. Freshly isolated lymph node cells (LNC)s or magnetically LNC-purified CD4+ T cells (Milteny beads) from naive (n=14) or immunized (n=5 for each time point) C57B1/6 mice were used to examine *ex vivo* mRNA expression of PK-2, PK-R1 and PK-R2. Total RNA was isolated from LNCs or magnetically purified (Miltenyi) CD4+ T cells by using Tri Reagent Solution (Ambion) according to the manufacturer's protocol. 1 µg RNA was reverse transcribed to cDNA with Quantitect® Reverse Transcription Kit, which includes a DNA removal step (Qiagen). Real Time PCR was performed on 7500 Fast Real-time PCR system (Applied Biosystems) by using Taqman® Fast Universal PCR Master Mix and TaqMan® gene expression assays for PK-2 (Mm01182450_g1), PK-R1 (Mm00517546_m1), PK-R2 (Mm03049046_m1) normalized on GAPDH (Mm99999915_g1) according to the manufacturer's protocol (Applied Biosystems). Data were analyzed by using the comparative Ct method and are expressed as the percentage of the housekeeping gene GAPDH (mean ± SD). The results are representative of two consecutive experiments that gave similar results. These results show an increase of mRNA expression of PK-2 by lymph node cells and purified CD4+ T cells in C57B1/6 mice during the disease. This increase of mRNA expression started before the onset of the disease reached its highest level at the onset and was less significant after the onset of EAE. This modulation of the expression of PK-2 was accompanied by a dramatic reduction of PK-R2 expression during chronic EAE. On the other hand, the expression of PK-R1 was not significantly modulated during the disease. Thus, the couple PK-2/PK-R1 seemed to play an important role during EAE, the experimental model of MS.

### Modulation of the levels of Prokineticin-2 in the serum of mice with MOG35-55 induced EAE (chronic EAE).

With reference to figure 3, the chronic EAE had been induced in C57B1/6 mice by immunization with MOG₃₅₋₅₅ (100 µg/mouse) and PTX (400 ng/mouse). Mice were sacrificed at different time points after the immunization and the blood serums prepared (5-6 mice at each time point). Prokineticin-2 levels in the serum were evaluated using an Elisa kit for murine Prokineticin-2 according to the manufacturer's protocol (Uscn, Life Science Inc., USA). Data are expressed as mean ng/ml ± SEM. *P* by two-tailed unpaired Student's t test vs. Naive mice.

These results show an important increase of the levels of the protein PK-2 in the serum of mice with MOG₃₅₋₅₅ induced EAE, suggesting an important role for this protein in EAE.

### Reduction in the severity of relapsing-remitting EAE in SJL mice treated with PC1

With reference to figure 4, the non-peptide prokineticin receptor 1 and 2 antagonist PC1 was administered intraperitoneally to 8-12 week old female SJL mice in which EAE had been induced by immunization with PLP₁₃₉₋₁₅₁ (100 µg/mouse) in CFA. The PC1 was given at a dose of 500 µg /kg (n=10). As a control, an equal amount of saline solution was injected (vehicle) (n=10). The treatments began at the time of induction of EAE (preventive treatment), or, more specifically, 4 hours after the induction of EAE. Ten mice per group were used. The graph represents the average daily disease score ± standard error of the mean (SEM). A scale of 0 to 5 was used to attribute the score, where 0 is the absence of symptoms and 5 is death (as described in Pedotti et al, Nat Immunol 2001). * *p*<0.05 for two-tailed unpaired Student's t test vs Control. The results are representative of three consecutive experiments that gave similar results.

Table 1 below shows the treatment of mice affected by relapsing-remitting EAE with the non-peptide prokineticin receptor 1 and 2 antagonist PC1, carried out as described in Figure 4. * *p*<0.05 for two-tailed unpaired Student's t test vs Control.

**Table 1. Treatment of EAE-affected mice with the non-peptide prokineticin receptor 1 and 2 antagonist PC-1, carried out as described in Figure 2. * p<0.05 for two-tailed unpaired Student's t test vs Control.**

| **Treatment** | **Incidence (%)** | **Onset of disease (Day)** | **Peak of disease Severity** | **Cumulative disease score** |
|---|---|---|---|---|
| Control | 100% (10/10) | 9.7 ± 0.3 | 3.0 ± 0.4 | 45.5 ± 10.1 |
| PC1 (500µg/kg) | 70% (7/10) | 12.4 ± 1.0 * | 1.7 ± 0.4* | 24.8 ± 9.0 * |

These results show that treating mice affected by relapsing-remitting EAE with PC1 induces a reduction in the severity of the disease, with a delay in the onset of the disease in mice treated from the time of induction of EAE and a reduction in the average severity at the peak of disease.

### Reduced T-cell response in splenocytes of mice with relapsing-remitting EAE treated with PC-1 in a preventive treatment regimen.

With reference to figure 5, 8-12 week old female SJL mice in which EAE had been induced by immunization with PLP₁₃₉₋₁₅₁ in CFA were preventively treated (day 0) with PC1 at a dose of 500 µg/kg per day, or with vehicle as described in Figure 4. The splenocytes were obtained 35 days after immunization with PLP₁₃₉₋₁₅₁, and were restimulated in vitro with the antigen PLP₁₃₉₋₁₅₁ at different concentrations or with the medium.

To assay proliferation, after 48 hours of incubation with 5% Co2 at 37°C, the cultures were loaded for 18 hours with 0.5 µCi 3H-thymidine per well. To assay cytokine production, splenocytes were restimulated in vitro with the antigen PLP₁₃₉₋₁₅₁ at different concentrations or with the medium. The concentration of Interferon-gamma (IFN-gamma), tumour necrosis factor (TNF), Interleukine-6 (IL-6), IL-17, IL-4, and IL-10 was determined in duplicate using the ELISA technique (ELISA DuoSet, Pharmingen, CA). The results are representative of two consecutive experiments that gave similar results.

The results show that in T lymphocytes stimulated with the myelin-specific antigen PLP₁₃₉₋₁₅₁ the treatment with the antagonist PC-1 brings about a reduction in peptide-specific T cells proliferation and in the production of inflammatory Th1 cytokines, which are very important for the development of EAE (IFN-gamma, and TNF), while increasing that of IL-4, the anti-inflammatory T-helper 2 cytokine.

### Reduction in the severity of relapsing-remitting EAE in SJL mice treated with the antagonist PC-7, administered in a preventive treatment regimen.

With reference to figure 6, the non-peptide prokineticin receptor 1 and 2 antagonist PC-7 was administered intraperitoneally to 8-12 week old female SJL mice in which EAE had been induced by immunization with PLP₁₃₉₋₁₅₁ (100 µg/mouse) in CFA. The PC7 was given at a dose of 500 µg /kg (n=10). As a control, an equal amount of saline solution was injected (vehicle) (n=10). The treatments began at the time of induction of EAE (preventive treatment), or, more specifically, 4 hours after the induction of EAE. Ten mice per group were used. The graph represents the average daily disease score ± standard error of the mean (SEM). The results are representative of three consecutive experiments that gave similar results.

Table 2 below shows the treatment of mice affected by relapsing-remitting EAE with the non-peptide prokineticin receptor 1 and 2 antagonist PC-7, carried out as described in Figure 6. * p<0.05 for two-tailed unpaired Student's t test vs Control.

**Table 2. Treatment of EAE-affected mice with the non-peptide prokineticin receptor 1 and 2 antagonist PC-7, carried out as described in Figure 5. * p<0.05 for two-tailed unpaired Student's t test.**

| **Treatment** | **Incidence (%)** | **Onset of disease (Day)** | **Peak in disease Severity** | **Cumulative disease score** |
|---|---|---|---|---|
| Control | 100% (10/10) | 10.4 ± 0.3 | 3.5 ± 0.3 | 45.1 ± 8.7 |
| PC7 (500µg/kg) | 90% (9/10) | 11.4 ± 0.1 * | 2.2 ± 0.3 * | 21.2 ± 5.1 * |

These results show that preventively treating mice affected by relapsing-remitting EAE with PC7 at a dose of 500µg/kg significantly reduces the severity of the disease.

### Reduction in the severity of relapsing-remitting EAE in SJL mice treated with the antagonist PC-7, administered in a therapeutic treatment regimen.

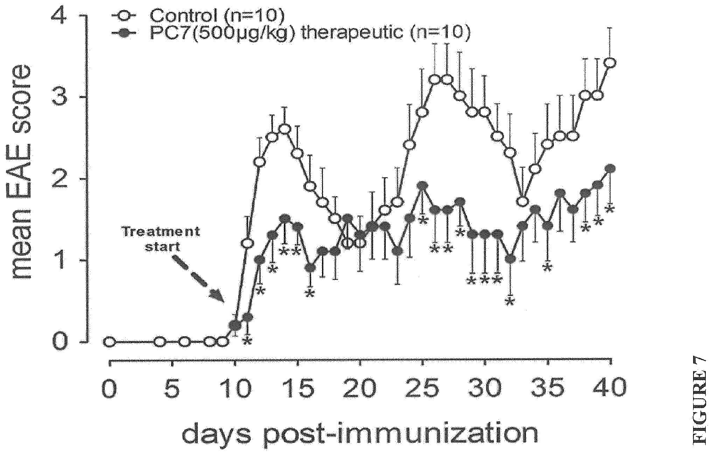

With reference to figure 7, the non-peptide prokineticin receptor 1 and 2 antagonist PC-7 was administered intraperitoneally to 8-12 week old female SJL mice in which EAE had been induced by immunization with PLP₁₃₉₋₁₅₁ (100 µg/mouse) in CFA. The PC-7 was given at a dose of 500 µg /kg (n=10). As a control, an equal amount of saline solution was injected (control) (n=10). The treatments began on the appearance of the first symptoms of the disease (Day 11). Ten mice per group were used. The graph represents the average daily disease score ± standard error of the mean (SEM). The results are representative of three consecutive experiments that gave similar results.

Table 3 below shows the treatment of mice affected by relapsing-remitting EAE with the non-peptide prokineticin receptor 1 and 2 antagonist PC-7, carried out as described in Figure 7. * p<0.05 for two-tailed unpaired Student's t test vs Control.

**Table 4. Treatment of EAE-affected mice with the non-peptide prokineticin receptor 1 and 2 antagonist PC-7, carried out as described in Figure 7. * p<0.05 for two-tailed unpaired Student's t test vs Control.**

| **Treatment** | **Incidence (%)** | **Peak in disease Severity** | **Cumulative disease score** |
|---|---|---|---|
| Placebo | 100% (10/10) | 3.4 ± 0.3 | 69.6 ± 9.8 |
| PC7 (500µg/kg) | 100% (10/10) | 2.6 ± 0.3 * | 42.3 ± 7.5 |

These results show that the therapeutically treating mice affected by relapsing-remitting EAE with PC7 at a dose of 500µg/kg starting from the appearance of the first symptoms of the disease significantly reduces the severity of the disease.

### Reduced T-cell response in the lymph nodes of mice with relapsing-remitting EAE treated with PC7 in a preventive treatment regimen

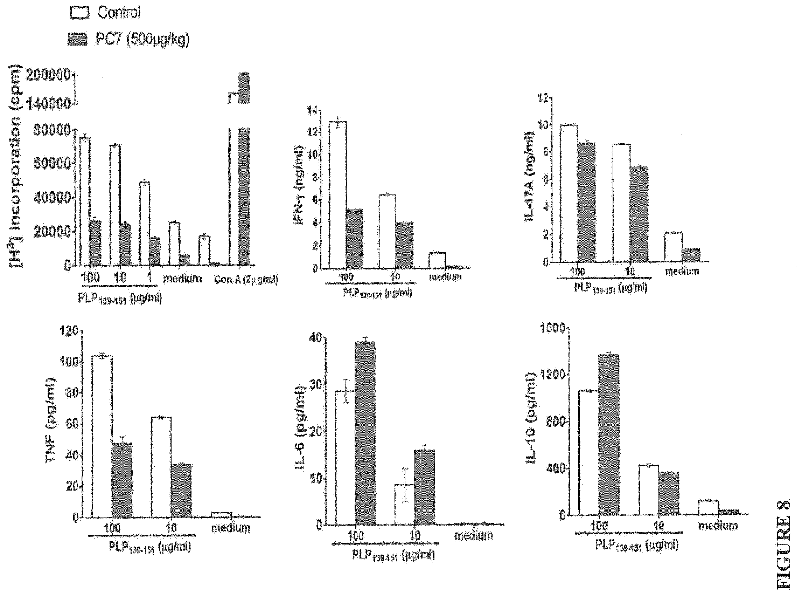

With reference to figure 8, 8-12 week old female SJL mice in which EAE had been induced by immunization with PLP₁₃₉₋₁₅₁ in CFA were preventively treated (day 0) with PC-7 at a dose of 500 µg/kg per day or with vehicle as described in the previous experiments. The draining lymph nodes (DLN, inguinal and axillary) were obtained 7 days after immunization with PLP₁₃₉₋₁₅₁. To assay proliferation, the DLN cells were restimulated in vitro with the antigen PLP₁₃₉₋₁₅₁ at different concentrations, with Concanavalin A (2 µg /ml), or with the medium.

After 48 hours of incubation with 5% Co2 at 37°C, the cultures were loaded for 18 hours with 0.5 µCi 3H-thymidine per well.

To assay cytokine production, DLN cells were restimulated *in vitro* with the antigen PLP₁₃₉₋₁₅₁ at different concentrations or with the medium. Data are expressed as mean cpm ± SEM, ng/ml ± SEM, or pg/ml ± SEM.

The results obtained show that the cells of the lymph nodes obtained from mice treated with PC-7 exhibit reduced proliferation and produce a significantly lower number of pro-inflammatory cytokines, such as IFN and TNF, when restimulated with PLP₁₃₉₋₁₅₁ compared to cells derived from mice treated with the vehicle (Control group). A slight reduction can also be observed in the levels of IL-17A. Moreover, an increase in the levels of interleukin IL-6 and of the anti-inflammatory interleukin IL-10 can be observed in cells derived from mice treated with PC-7, compared to cells derived from mice treated only with the vehicle (Control).

### Reduction in the severity of chronic EAE in C57B1/6 mice treated with the prokineticin receptor antagonist PC-7, administered in a preventive treatment regimen.

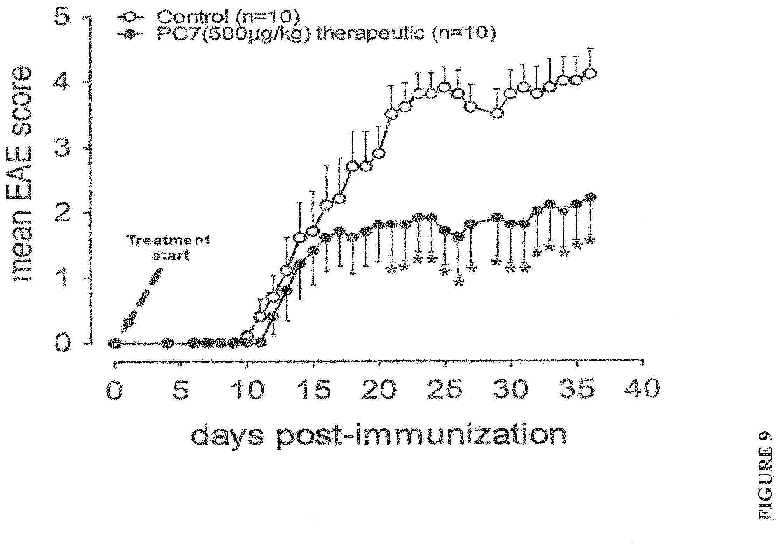

With reference to figure 9, the non-peptide prokineticin receptor 1 and 2 antagonist PC7 was administered intraperitoneally to 8-12 week old female C57B1/6 mice in which chronic EAE had been induced by immunization with MOG₃₅₋₅₅ (100 µg/mouse) and PTX (400 ng/mouse). The PC-7 was given at a dose of 500 µg /kg (n=10). As a control, an equal amount of saline solution was injected (vehicle) (n=10). The treatments began at the time of induction of EAE (preventive treatment), or, more specifically, 4 hours after the induction of EAE. Ten mice per group were used. The graph represents the average daily disease score ± standard error of the mean (SEM). The results are representative of three consecutive experiments that gave similar results.

Table 4 below shows the treatment of mice affected by chronic EAE with the non-peptide antagonist of the prokineticin receptors 1 and 2 PC7, carried out as described in Figure 9. * p<0.05 for two-tailed unpaired Student's t test vs Control.

**Table 5. Treatment of mice affected by chronic EAE with the non-peptide antagonist of the prokineticin receptors 1 and 2 PC-7, carried out as described in Figure 9. * p<0.05 for two-tailed unpaired Student's t test vs Control.**

| **Treatment** | **Incidence (%)** | **Onset of disease (Day)** | **Peak in disease Severity** | **Cumulative disease score** |
|---|---|---|---|---|
| Control | 100% (10/10) | 12.3 ± 0.4 | 4.5 ± 0.2 | 77.1 ± 6.2 |
| PC7 (500µg/kg) | 90% (9/10) | 16.8 ± 1.7 * | 2.5 ± 0.5 * | 41.1 ± 11.8 * |

The results obtained show that the preventive treatment with PC7 induces a significant reduction in the severity of the disease.

### Reduction in the severity of chronic EAE in C57B1/6 mice treated with the prokineticin receptor antagonist PC7, administered in a therapeutic treatment regimen.

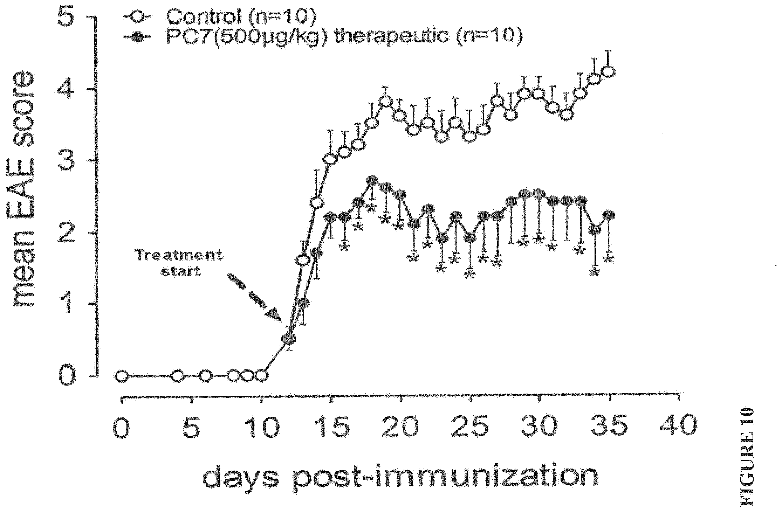

With reference to figure 10, the non-peptide prokineticin receptor 1 and 2 antagonist PC-7 was administered intraperitoneally to 8-12 week old female C57B1/6 mice in which chronic EAE had been induced by immunization with MOG₃₅₋₅₅ (100 µg/mouse) and PTX (400 ng/mouse). The PC-7 was given at a dose of 500 µg /kg (n=10). As a control, an equal amount of saline solution was injected (vehicle) (n=10). The treatments began on the onset of the first symptoms of the disease (day 12). Ten mice per group were used. The graph represents the average daily disease score ± standard error of the mean (SEM). The results are representative of two consecutive experiments that gave similar results.

Table 5 below shows the treatment of mice affected by chronic EAE with the non-peptide prokineticin receptor 1 and 2 antagonist PC-7, carried out as described in Figure 10. * p<0.05 for two-tailed unpaired Student's t test vs Control.

**Table 6. Treatment of mice affected by chronic EAE with the non-peptide prokineticin receptor 1 and 2 antagonist PC-7, carried out as described in Figure 10. * p<0.05 for two-tailed unpaired Student's t test vs Control.**

| **Treatment** | **Incidence (%)** | **Peak in disease Severity** | **Cumulative disease score** |
|---|---|---|---|
| Control | 100% (10/10) | 4.4 ± 0.2 | 79.8 ± 5.0 |
| PC7 (500µg/kg) | 100% (10/10) | 3.3 ± 0.3 * | 50.9 ± 11.8 * |

The results obtained show that the therapeutic treatment with PC7 at a dose of 500 µg/Kg induces a significant reduction in the severity of the disease in chronic EAE.

### Reduced T-cell response in the lymph nodes of mice with chronic EAE treated with PC-7 in a preventive treatment regimen.

With reference to figure 11, 8-12 week old female C57B1/6 mice in which chronic EAE had been induced by immunization with MOG₃₅₋₅₅ were preventively treated (day 0) with PC-7 at a dose of 500 µg/kg per day or with a vehicle as described in the previous experiments. The draining lymph nodes (DLN, inguinal and axillary) were obtained 10 days after immunization with MOG₃₅₋₅₅. To assay proliferation, the DLN cells were restimulated in vitro with MOG₃₅₋₅₅ at different concentrations, with Concanavalin A (2 µg /ml), or with the medium. Data are expressed as mean cpm ± SEM, ng/ml ± SEM, or pg/ml ± SEM.

After 48 hours of incubation with 5% Co2 at 37°C, the cultures were loaded for 18 hours with 0.5 µCi 3H-thymidine per well.

To assay cytokine production, DLN cells were restimulated in vitro with MOG₃₅₋₅₅ at different concentrations or with the medium.

The results obtained show that in T lymphocytes stimulated with the myelin-specific antigen MOG₃₅₋₅₅ the treatment with the antagonist PC-7 induces a reduction in peptide-specific T cells proliferation and in the production of the pro-inflammatory Th1 and thl7 cytokines, which are very important for the development of EAE (IFN-gamma, TNF and IL-17), while increasing that of the suppressor IL-10, the anti-inflammatory T-h2 cytokine.

It was thus demonstrated that prokineticins and their receptors seem to be involved in the physiopathology of two models of EAE, the experimental model of human multiple sclerosis, and that the use of non-peptide prokineticin receptor antagonists in accordance with the invention shows particular effectiveness in the treatment of autoimmune diseases or diseases with an autoimmune component, or dysimmune diseases or diseases with a dysimmune component affecting the central nervous system, peripheral nervous system and neuromuscular junction.

### Main references:

1 Negri, L., R. Lattanzi, E. Giannini, and P. Melchiorri. 2007. Bv8/Prokineticin proteins and their receptors. Life Sci 81:1103-1116.
2 Martucci, C., S. Franchi, E. Giannini, H. Tian, P. Melchiorri, L. Negri, and P. Sacerdote. 2006. Bv8, the amphibian homologue of the mammalian prokineticins, induces a proinflammatory phenotype of mouse macrophages. Br J Pharmacol 147:225-234
3 Franchi, S., E. Giannini, D. Lattuada, R. Lattanzi, H. Tian, P. Melchiorri, L. Negri, A. E. Panerai, and P. Sacerdote. 2008. The prokineticin receptor agonist Bv8 decreases IL-10 and IL-4 production in mice splenocytes by activating prokineticin receptor-1. BMC Immunol 9:60.
4 Steinman, L. 2008. A rush to judgment on Th17. The Journal of experimental medicine 205:1517-1522.
5 Liblau, R. S., S. M. Singer, and H. O. McDevitt. 1995. Th1 and Th2 CD4+ T cells in the pathogenesis of organ-specific autoimmune diseases. Immunology today 16:34-38.
6 Pedotti, R., D. Mitchell, J. Wedemeyer, M. Karpuj, D. Chabas, E. M. Hattab, M. Tsai, S. J. Galli, and L. Steinman. 2001. An unexpected version of horror autotoxicus: anaphylactic shock to a self-peptide. Nat Immunol 2:216-222.
7 Pedotti, R., Jason J. DeVoss, S. Youssef, D. Mitchell, J. Wedemeyer, r. Madanat, H. Garren, P. Fontoura, M. Tsai, S. Galli, R. Sobel, and L. Steinman. 2003. Multiple elements of the allergic arm of the immune response modulate autoimmune demyelination. PNAS Vol.100, no.4, 1867-1872
8 Urayama, K., D.B. Dedeoglu, C.Guillini, S. Frantz, G. Ertl, N. Messaddeq and C.G. Nebigil 2009. Transgenic myocardial overexpression of prokineticin receptor-2 (GPR73b) induces hypertrophy and capillary vessel leakage. Cardiovasc Res 81:28-37.
9 Guilini, C., K. Urayama, G. Turkeri, D. B. Dedeoglu, H. Kurose, N. Messaddeq, and C. G. Nebigil. 2010. Divergent roles of prokineticin receptors in the endothelial cells: angiogenesis and fenestration. Am J Physiol Heart Circ Physiol 298:H844-852.

## Claims

1. Compound selected from non-peptide prokineticin receptors antagonists of chemical structure (PC1) or (PC7) 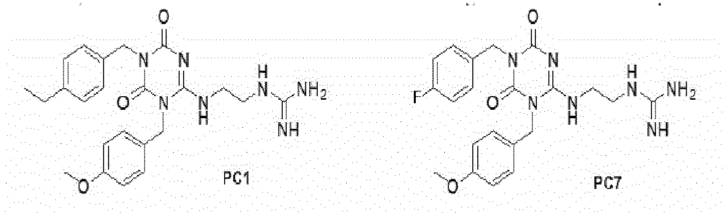 for use in the treatment of immune-mediated diseases of the central nervous system, peripheral nervous system and neuromuscular junction.

2. Compound for use according to claim 1 for modulating, directly and indirectly, the specific autoimmune response against myelin by shifting it from a T-helper 1-type phenotype to a T-helper 2-type phenotype so as to reduce the production of T-helper 1 inflammatory cytokines and increase the production of T-helper 2-type inflammatory cytokines.

3. Compound selected from non-peptide prokineticin receptors antagonists according to any one of the previous claim, for use in the treatment of human multiple sclerosis and/or experimental autoimmune encephalomyelitis (EAE).

4. Compound according to claim 3 for use in the treatment of relapsing-remitting experimental autoimmune encephalomyelitis (EAE).

5. Compound according to claim 3 for use in the treatment of chronic experimental autoimmune encephalomyelitis (EAE).

## Patentansprüche

1. Verbindung, ausgewählt aus Nicht-Peptid-Prokineticin-Rezeptor-Antagonisten der chemischen Struktur (PC1) oder (PC7) 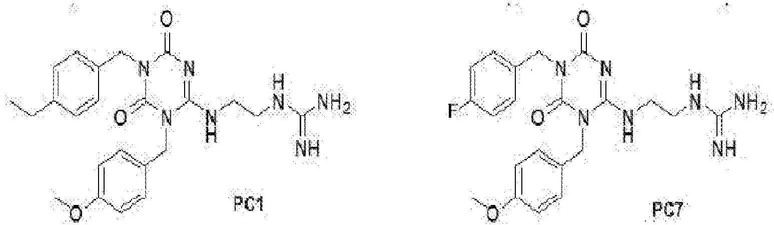 zur Verwendung bei der Behandlung von immunvermittelten Erkrankungen des Zentralnervensystems, des peripheren Nervensystems und der neuromuskulären Synapse.

2. Verbindung zur Verwendung nach Anspruch 1 zur direkten und indirekten Modulation der spezifischen Autoimmunantwort gegen Myelin durch Verschiebung von einem T-Helfer-Phänotyp Typ-1 zu einem T-Helfer-Phänotyp Typ 2, um so die Produktion von T-Helfer-1 inflammatorischen Zytokinen zu verringern und die Produktion von inflammatorischen Zytokinen vom T-Helfer-2-Typ zu erhöhen.

3. Verbindung, die ausgewählt ist aus Nicht-Peptid-Prokineticin-Rezeptor-Antagonisten nach einem der vorhergehenden Ansprüche, zur Verwendung bei der Behandlung von Multipler Sklerose und/oder experimenteller Autoimmun-Enzephalomyelitis (EAE) des Menschen.

4. Verbindung nach Anspruch 3 zur Verwendung bei der Behandlung von schubweise remittierend verlaufender experimenteller Autoimmun-Enzephalomyelitis (EAE).

5. Verbindung nach Anspruch 3 zur Verwendung bei der Behandlung von chronischer experimenteller Autoimmun-Enzephalomyelitis (EAE).

## Revendications

1. Composé choisi parmi les antagonistes non peptidiques de récepteurs de prokinéticine de structure chimique (PC1) ou (PC7) 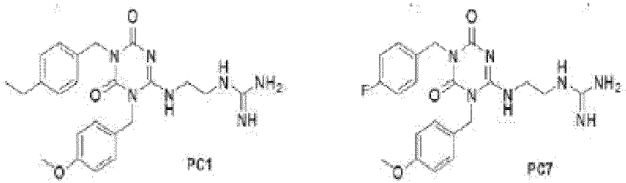 pour l'utilisation dans le traitement de maladies immunitaires du système nerveux central, du système nerveux périphérique et des jonctions neuromusculaires.

2. Composé pour une utilisation selon la revendication 1 pour moduler, directement et indirectement, la réponse auto-immune spécifique contre la myéline en la faisant passer du phénotype lymphocyte T auxiliaire de type 1 au phénotype lymphocyte T auxiliaire de type 2 de manière à réduire la production de cytokines de l'inflammation de lymphocyte auxiliaire 1 et à augmenter la production de cytokines de l'inflammation de lymphocytes auxiliaires de type 2.

3. Compose choisi parmi les antagonistes non peptidiques de récepteurs de prokinéticine selon l'une quelconque des revendications précédentes pour une utilisation dans le traitement de la sclérose en plaques humaine et/ou l'encéphalomyélite auto-immune expérimentale (EAE).

4. Composé selon la revendication 3 pour une utilisation dans le traitement de rechute-rémission de l'encéphalomyélite auto-immune expérimentale (EAE).

5. Composé selon la revendication 3 pour une utilisation dans le traitement de l'encéphalomyélite chronique auto-immune expérimentale (EAE).
